# EUROPEAN PATENT SPECIFICATION

(11) **EP 1 671 649 B1**
(45) Date of publication and mention of the grant of the patent: **23.11.2011**
(21) Application number: 04791942.8
(22) Date of filing: 01.10.2004
(51) Int. Cl.: A61K 45/00, A61K 31/472, A61P 3/10, A61P 5/50, C07D 217/24

(54) **Dipeptidyl peptidase IV inhibitors for treating diabetic patients with sulfonylurea secondary failure**
Dipeptidylpeptidase-IV-Inhibitoren zur Behandlung von Diabetes-Patienten mit Sekundärversagen durch Sulfonylharnstoffe
Inhibiteurs de la dipeptidyl peptidase IV pour le traitement de patients diabétiques souffrant d'une défaillance secondaire dûe aux sulfonylurées

(30) Priority: 03.10.2003 JP 2003345740
(43) Date of publication of application: 21.06.2006
(73) Proprietor: Takeda Pharmaceutical Company Limited, Osaka-shi, Osaka 541-0045 (JP)
(72) Inventor: ASAKAWA, Tomoko c/o Takeda Pharmaceut. Comp. Ltd., Osaka-shi, Osaka (JP)
(74) Representative: Dossmann, Gérard
(86) International application number: PCT/JP2004/014475
(87) International publication number: WO 2005/032590

(56) References cited:
- EP-A- 1 422 293
- WO-A-2004/024184
- JP-A- 2003 238 566
- US-A1- 2003 073 728
- KIYONO H. ET AL: 'Sui beta ATP Kanjusei K+ Channel to Sulfonylured Receptor no Kino Kyokan no Kaiseki, Oyobi Atarashii Sayo Kijo ni yoru Keiko Tonyobyoyaku no Kaihatsu' RESEARCH PAPERS OF THE SUZUKEN MEMORIAL FOUNDATION 1999, SUZUKEN MEMORIAL FOUNDATION 2001, pages 147 - 149, XP002995816
- FANGHANEL G. ET AL: 'Metformin's Effects on Glucose and Lipid Metabolism in Patients with Secondary Failure to Sulfonylureas' DIABETES CARE vol. 19, no. 11, 1996, pages 1185 - 1189, XP002995817
- YAMADA K. ET AL: 'Keiko Yakubutsu Ryoho no Shinsenryaku 2. Keiko Ketto Tokayaku no Tsukaiwake to Heiyo' TONYOBYOGAKU NO SHINPO vol. 36, 2002, pages 292 - 296, XP002995818
- TRISCHITTA V. ET AL: 'Comparison of Combined Therapies in Treatment of Secondary Failure to Glyburide' DIABETES CARE vol. 15, no. 4, 1992, pages 539 - 542, XP002995819
- KITAHARA Y. ET AL: 'Tonyobyo Ippan ni Kansuru Chiryoyaku- Sokkogata Insulin Bunpitsu Sokushinzai ni yoru Seiritekina To no Nagare ´no Jitsugen' SAIBO vol. 32, no. 12, 2000, pages 480 - 483, XP002995820
- 'SU-zai ni 2ji Muko to Kangaerareru 2gata Tonyobyo Kanja ni okeru Chosokkogata Insulin Bunpitsu Sokushinzai nateglinide (Na) no Koka' THE JOURNAL OF THE JAPAN DIABETIC SOCIETY vol. 43, no. 1, 2000, page S-120, XP002995821
- '2gata Tonyobyo Kanja no Sulfonylurea-zai 2ji Mukorei ni Taisuru Sokkogata Shokugo Ketto Tokazai (Nateglinide) no Shiyo Keiken' NIPPON TAISHITSUGAKU ZASSHI vol. 63, no. 1-2, 2001, page 52, XP002995822
- BORISSOVA A-M ET AL: "FACTORS FOR DEVELOPMENT OF SECONDARY FAILURE TO SULFONYLUREA DRUGS IN NON-INSULIN-DEPENDENT DIABETES MELLITUS", ACTA DIABETOLOGICA LATINA, vol. 28, no. 1, 1991, pages 91-98, ISSN: 0001-5563
- HERRMANN-RINKE C ET AL: "Regulation of glucagon-like peptide-1 secretion from rat ileum by neurotransmitters and peptides", JOURNAL OF ENDOCRINOLOGY, vol. 147, no. 1, 1995, pages 25-31, ISSN: 0022-0795

## Description

### Technical Field

The present invention relates to an agent for treating diabetes with sulfonylurea secondary failure which comprises a dipeptidyl peptidase IV (hereinafter sometimes referred to as DPP-IV) inhibitor.

### Background Art

A sulfonylurea compound (hereinafter sometimes referred to as an SU agent) has been generally used as a first-choice oral hypoglycemic agent. Repeated administration of an SU agent to a diabetic patient, however, may cause the patient a condition in which the agent is ineffective in lowering the blood sugar level, namely sulfonylurea secondary failure.

Diabetic patients with sulfonylurea secondary failure are treated with insulin preparations because administration of SU agents is not expected to make therapeutic effect on them.

DPP-IV inhibitors are known to be useful as agents for treating diabetes (see, for example, International Publication No.WO02/062764 Pamphlet).

### Disclosure of Invention

### Problems to be solved by the Invention

An object of the present invention is to provide an agent for treating diabetes with sulfonylurea secondary failure showing excellent insulin-secreting and hypoglycemic effects on even diabetic patients on whom a sulfonylurea compound or a fast-acting insulin secretagogue has no insulin-secreting effect and therefore no sufficient hypoglycemic effect.

Another object of the present invention is to provide an agent for treating diabetes with sulfonylurea secondary failure having no side effect such as vascular complications and hypoglycemia which are caused by administration (especially long-term administration) of an insulin preparation to diabetic patients with sulfonylurea secondary failure and showing excellent therapeutic effect.

### Means for Solving the Problem

As the result of intensive study, the present inventor found for the first time that a DPP-IV inhibitor was useful as an agent for treating diabetes with sulfonylurea secondary failure, and then completed the present invention.

That is, the present invention relates to:
1) an agent for treating diabetes with sulfonylurea secondary failure which comprises a dipeptidyl peptidase IV inhibitor;
2) the agent according to the above 1) wherein the sulfonylurea secondary failure is ascribable to a sulfonylurea compound;
3) the agent according to the above 1) wherein the sulfonylurea secondary failure is ascribable to a fast-acting insulin secretagogue;
4) use of a dipeptidyl peptidase IV inhibitor for manufacture of an agent for treating diabetes with sulfonylurea secondary failure;
5) a method of treating diabetes with sulfonylurea secondary failure in a mammal which comprises administering an effective amount of a dipeptidyl peptidase IV inhibitor to the mammal;
6) an insulin secretagogue for diabetic patients with sulfonylurea secondary failure which comprises a dipeptidyl peptidase IV inhibitor;
7) use of a dipeptidyl peptidase IV inhibitor for manufacture of an insulin secretagogue for diabetic patients with sulfonylurea secondary failure;
8) a method of promoting insulin secretion in a diabetic patient with sulfonylurea secondary failure which comprises administering an effective amount of a dipeptidyl peptidase IV inhibitor to the patient; and the like.

### Effect of the Invention

The agent for treating diabetes with sulfonylurea secondary failure of the present invention shows excellent insulin-secreting and hypoglycemic effects on even diabetic patients on whom a sulfonylurea compound or a fast-acting insulin secretagogue has no insulin-secreting effect and therefore no sufficient hypoglycemic effect.

The agent for treating diabetes with sulfonylurea secondary failure of the present invention can be used safely without side effect (e.g. vascular complication, hypoglycemia) caused by administration (especially long-term administration) of an insulin preparation and side effect (e.g. hypoglycemia, vomiting) caused by administration of a glucagon-like peptide (GLP)-1.

### Best Mode for Carrying Out the Invention

In the specification, a DPP-IV inhibitor means a compound that inhibits the enzyme activity of DPP-IV [EC3.4.14.5 according to classification by the Committee for Nomenclature, International Union of Biochemistry and Molecular Biology (IUBMB)]. The compound may be peptidic or nonpeptidic, and it is preferably nonpeptidic.

The form of a DPP-IV inhibitor may be different between before and after administration into the body as long as the DPP-IV inhibiting activity is retained. That is, a DPP-IV inhibitor may be an "active metabolite" having the DPP-IV inhibiting activity after the DPP-IV inhibitor is metabolized in vivo to a substance with a different structure. In addition, a DPP-IV inhibitor may be a "prodrug" that is changed into an active substance as a result of reaction with an enzyme, gastric acid, etc. under the physiological conditions in vivo.

The DPP-IV inhibiting activity can be confirmed with a method utilizing "the method of Raymond et al. (Diabetes, Vol.47, pp.1253-1258, 1998)".

DPP-IV inhibitors include nitrogen-containing heterocyclic compounds, specifically, the following compounds (1) to (13).

### (1) A compound of the formula:

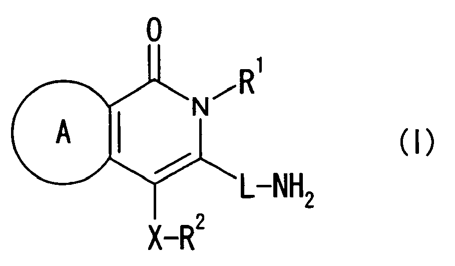

wherein, the ring A is an optionally substituted 5 to 10-membered aromatic ring,
R¹ and R² are the same or different and are independently an optionally substituted hydrocarbon group or an optionally substituted heterocyclic group,
X is a bond, -O-, -S-, -SO-, -SO₂- or -NR³- (wherein R³ is a hydrogen atom or an optionally substituted hydrocarbon group), and
L is a divalent hydrocarbon group, or a salt thereof, as disclosed in WO02/062764.

A salt of the compound of the formula (I) is preferably a pharmacologically acceptable salt. Such a salt includes, for example, salts with inorganic bases, salts with organic bases, salts with inorganic acids, salts with organic acids, and salts with basic or acidic amino acids.

Preferred examples of salts with inorganic bases include salts with alkali metals such as sodium and potassium; alkaline earth metals such as calcium and magnesium; aluminum, ammonium and the like.

Preferred examples of salts with organic bases include salts with trimethylamine, triethylamine, pyridine, picoline, ethanolamine, diethanolamine, triethanolamine, dicyclohexylamine, N,N-dibenzylethylenediamine and the like.

Preferred examples of salts with inorganic acids include salts with hydrochloric acid, hydrobromic acid, nitric acid, sulfuric acid, phosphoric acid and the like.

Preferred examples of salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferred examples of salts with basic amino acids include salts with arginine, lysine, ornithine and the like.

Preferred examples of salts with acidic amino acids include salts with aspartic acid, glutamic acid and the like.

The compound of the formula (I) may be anhydrous or hydrous, and further may be a prodrug.

Preferred examples of the compound of the formula (I) include the following compounds.

### (Compound I-a)

A compound wherein the ring A is a benzene ring optionally substituted with 1 or 2 substituents selected from
1) cyano;
2) C₁₋₁₀ alkyl (preferably ethyl) or C₂₋₁₀ alkenyl (preferably ethenyl), each of which may be optionally substituted with carbamoyl or carboxyl;
3) optionally substituted hydroxyl [preferably, C₁₋₁₀alkoxy (preferably methoxy, isopropoxy) optionally substituted with 1 to 3 substituents selected from carbamoyl, carboxyl and C₂₋₅ alkoxycarbonyl (preferably methoxycarbonyl); hydroxyl; C₇₋₁₃ aralkyloxy (preferably benzyloxy)] [more preferably, carbamoylmethoxy];
4) acyl [preferably, C₁₋₆ alkyl-carbonyl (preferably acetyl), carbamoyl, mono- or di-(C₁₋₆ alkyl optionally substituted with 1 to 3 substituents selected from halogen and C₁₋₆ alkoxy-carbonyl)-carbamoyl (preferably methylcarbamoyl, ethylcarbamoyl, propylcarbamoyl, dimethylcarbamoyl, trifluoroethylcarbamoyl, ethoxycarbonylmethylcarbamoyl and the like), C₃₋₁₀ cycloalkyl-carbamoyl (preferably cyclopropylcarbamoyl), C₇₋₁₃ aralkyl-carbamoyl (preferably benzylcarbamoyl), nitrogen-containing heterocyclic-carbonyl optionally substituted with hydroxy (preferably pyrrolidinylcarbonyl, piperidinocarbonyl), C₁₋₆ alkylsulfonyl (preferably methylsulfonyl), C₁₋₆ alkylsulfinyl (preferably methylsulfinyl), carboxyl, C₁₋₆ alkoxy-carbonyl (preferably methoxycarbonyl), thiocarbamoyl];
5) optionally substituted amino (preferably carbamoylamino);
6) optionally substituted thiol [preferably C₁₋₁₀alkylthio (preferably methylthio) optionally substituted with carbamoyl];
7) optionally substituted heterocyclic group [preferably, an aromatic heterocyclic group (preferably, furyl, thienyl, oxazolyl, oxadiazolyl, thiazolyl, tetrazolyl, pyridyl, pyrrolyl, triazolyl) or a nonaromatic heterocyclic group (preferably, dioxoisoindol, 5-oxooxadiazol-3-yl, 5-oxothiadiazol-3-yl), each of which may be optionally substituted with 1 or 2 substituents selected from C₁₋₆ alkyl optionally substituted with 1 to 3 halogen atoms (preferably methyl, trifluoromethyl), carboxyl, C₂₋₈ alkoxycarbonyl (preferably ethoxycarbonyl), cyano, carbamoyl, amino, mono- or di-C₂₋₁₀ alkanoylamino (e.g. acetylamino, isopentanoylamino), C₁₋₁₀ alkoxy-carbonylamino (e.g. methoxycarbonylamino), carbamoylamino, mono- or di-C₁₋₁₀ alkyl-carbamoylamino (e.g. methylcarbamoylamino, dimethylcarbamoylamino), C₆₋₁₄ aryl-carbonylamino (e.g. benzoylamino), C₃₋₁₀ cycloalkyl-carbonylamino, C₇₋₁₃ aralkyloxy-carbonylamino, mono- or di-C₁₋₁₀ alkylsulfonylamino (e.g. methylsulfonylamino, dimethylsulfonylamino), C₆₋₁₄ arylsulfonylamino and C₁₋₆ alkoxy-carbamoylamino (e.g. methoxycarbamoylamino)]; and 8) amidino;
   R¹ is C₄₋₁₀alkyl (preferably isobutyl, neopentyl) or C₄₋₁₀ cycloalkylalkyl (preferably cyclopropylmethyl);
   R² is a C₆₋₁₄ aryl (preferably phenyl) optionally substituted with 1 or 2 substituents selected from halogen (preferably fluorine, chlorine) and C₁₋₆ alkyl (preferably methyl);
   X is a bond; and
   L is C₁₋₁₀ alkylene (preferably -CH₂-).

### (Compound I-b)

A compound wherein the ring A is a benzene ring optionally substituted with 1 or 2 substituents selected from
1) C₁₋₁₀ alkyl (preferably ethyl) or C₂₋₁₀ alkenyl (preferably ethenyl), each of which may be optionally substituted with C₂₋₈ alkoxycarbonyl (preferably ethoxycarbonyl) or carbamoyl;
2) optionally substituted hydroxyl [preferably, C₁₋₁₀ alkoxy (preferably methoxy) optionally substituted with carbamoyl; more preferably carbamoylmethoxy];
3) acyl (preferably carbamoyl, thiocarbamoyl, carboxyl); and
4) optionally substituted heterocyclic group [preferably, an aromatic heterocyclic group (preferably, furyl, thienyl, oxazolyl, oxadiazolyl, thiazolyl, tetrazolyl, pyridyl, pyrrolyl, triazolyl) or a nonaromatic heterocyclic group (preferably, 5-oxooxadiazol-3-yl), each of which may be optionally substituted with 1 or 2 substituents selected from C₁₋₆ alkyl (preferably methyl), carboxyl, C₂₋₈ alkoxycarbonyl (preferably ethoxycarbonyl), cyano, carbamoyl, amino, mono- or di-C₂₋₁₀ alkanoylamino (e.g. acetylamino, isopentanoylamino), C₁₋₁₀ alkoxy-carbonylamino (e.g. methoxycarbonylamino), carbamoylamino, mono- or di-C₁₋₁₀ alkyl-carbamoylamino (e.g. methylcarbamoylamino, dimethylcarbamoylamino), C₆₋₁₄ aryl-carbonylamino (e.g. benzoylamino), C₃₋₁₀ cycloalkyl-carbonylamino, C₇₋₁₃ aralkyloxy-carbonylamino, mono- or di-C₁₋₁₀ alkylsulfonylamino (e.g. methylsulfonylamino, dimethylsulfonylamino), C₆₋₁₄ arylsulfonylamino and C₁₋₆ alkoxy-carbamoylamino (e.g. methoxycarbamoylamino)];
   R¹ is C₄₋₁₀ alkyl (preferably isobutyl, neopentyl) or C₄₋₁₀ cycloalkylalkyl (preferably cyclopropylmethyl);
   R² is C₁₋₁₀ alkyl (preferably butyl) optionally substituted with 1 to 3 halogen atoms;
   X is -O-; and
   L is C₁₋₁₀ alkylene (preferably -CH₂-).
   Among the compounds of the formula (I), particularly preferred are 2-[3-(aminomethyl)-4-butoxy-2-isobutyl-1-oxo-1,2-dihydro-6-isoquinolyl]-1,3-thiazol-4-carbonitrile;
   2-[3-(aminomethyl)-4-butoxy-2-isobutyl-1-oxo-1,2-dihydro-6-isoquinolyl]-1,3-thiazol-4-carboxylic acid;
   2-[3-(aminomethyl)-4-butoxy-2-isobutyl-1-oxo-1,2-dihydro-6-isoquinolyl]-1,3-thiazol-4-carboxamide;
   ethyl 2-[3-(aminomethyl)-4-butoxy-2-isobutyl-1-oxo-1,2-dihydro-6-isoquinolyl]-1,3-thiazol-4-carboxylate;
   (E)-3-[3-(aminomethyl)-4-butoxy-2-isobutyl-1-oxo-1,2-dihydro-6-isoquinolyl]-2-propenamide;
   (E)-3-[3-(aminomethyl)-2-isobutyl-4-phenyl-1-oxo-1,2-dihydro-6-isoquinolyl]-2-propenamide;
   3-(aminomethyl)-2-isobutyl-1-oxo-4-phenyl-1,2-dihydro-6-isoquinolinecarboxamide;
   2-{[3-(aminomethyl)-2-isobutyl-4-phenyl-1-oxo-1,2-dihydro-6-isoquinolyl]oxy}acetamide, and the like.

(2) A compound of the formula: 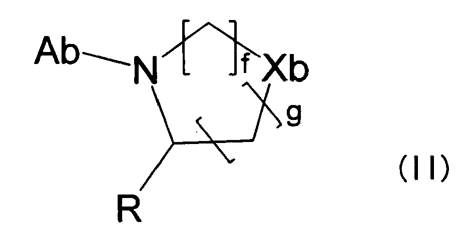 wherein, f is 1 or 2; g is 0, 1 or 2; Xb is -CH₂-, -O-, -S-, -SO-, -SO₂- or -NR³- (wherein R³ is hydrogen or C₁₋₆ alkyl);
   R is hydrogen, cyano, -CHO, -B(OH)₂-, -P(O)(OR³), -CCR⁴ or - CH=NR⁵ (wherein R⁴ is hydrogen, fluorine, C₁₋₆ alkyl, cyano, nitro, -OR³, -CO₂R³ or -COR³ (wherein R³ is as defined above); R⁵ is phenyl, hydroxyl, -OR³, -OCOR³ or benzyloxy (wherein R³ is as defined above)); and Ab is an optionally substituted amino acid residue, or a salt thereof, as disclosed in WO95/15309 etc.
   In the formula, the C₁₋₆ alkyl represented by R³ includes methyl, ethyl, propyl, isopropyl, butyl, isobutyl, sec-butyl, t-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, and the like.
   The amino acid residue of the "optionally substituted amino acid residue" represented by Ab includes a group after removing the OH of the constitutive carboxyl group from α-amino acid or β-amino acid.
   The α-amino acid includes alanine, arginine, asparagine, aspartic acid, glutamine, glutamic acid, glycine, histidine, isoleucine, leucine, lysine, methionine, phenylalanine, proline, serine, threonine, tryptophan, tyrosine, valine, citrulline, ornithine, homocysteine, and the like.
   The β-amino acid includes β-alanine, β-aminocyclopropanoic acid, β-aminocyclobutanoic acid, β-aminocyclopentanoic acid, β-aminocyclohexanoic acid, β-aminocycloheptanoic acid, β-aminocyclooctanoic acid, and the like. The β-amino acid may have an unsaturated bond in the carbon chain constituting the amino acid.
   The above-mentioned α-amino acid and β-amino acid may be of D-, L- or DL-form, and preferably of natural L-form.
   The above-mentioned amino acid residue may be optionally substituted with 1 or 2 substituents on an amino group constituting the amino acid or on the side chain of the amino acid.
   The above-mentioned "substituent on the amino group" is preferably an optionally substituted hydrocarbon group, an optionally substituted piperidinyl group, or the like.
   The hydrocarbon group of the "optionally substituted hydrocarbon group" includes C₁₋₆ alkyl, C₃₋₁₂ cycloalkyl, C₂₋₆ alkenyl, C₃₋₁₂ cycloalkenyl, C₂₋₆ alkynyl, C₄₋₁₂ cycloalkadienyl, C₆₋₁₄ aryl (preferably phenyl), C₇₋₁₅ aralkyl (preferably benzyl, phenethyl), adamantyl, bicyclo[2.2.1]heptyl, and bicyclo[3.1.1]heptyl.
   The hydrocarbon group may be optionally substituted with 1 to 3 substituents at substitutable positions, and such substituents include halogen (preferably fluorine, chlorine); cyano; hydroxyl optionally substituted with acyl; hydroxymethyl; C₁₋₆ alkoxy optionally substituted with 1 to 3 halogen atoms (preferably fluorine); and amino optionally mono- or di-substituted with optionally substituted C₆₋₁₄ aryl or an optionally substituted heterocyclic group.
   The acyl of "hydroxyl optionally substituted with acyl" includes the acyl exemplified above as substituents of the ring A in Compound I-a.
   The C₆₋₁₄ aryl of the "optionally substituted C₆₋₁₄ aryl" includes phenyl, naphthyl, and the like.
   The heterocyclic group of the "optionally substituted heterocyclic group" includes pyridyl, pyrimidyl, pirazyl, quinolyl, isoquinolyl, quinoxalyl, and the like.
   The C₆₋₁₄ aryl and the heterocyclic group may be optionally substituted with 1 to 3 substituents at substitutable positions, and such substituents include halogen (preferably fluorine, chlorine, bromine); cyano; nitro; C₁₋₆ alkyl; C₁₋₆ alkoxy optionally substituted with 1 to 3 halogen atoms (preferably fluorine); carboxyl; carbamoyl; C₁₋₆ alkylsulfonyl (preferably methanesulfonyl); aminosulfonyl optionally mono- or di-substituted with C₁₋₆ alkyl (preferably dimethylaminosulfonyl); and the like.
   A substituent for the above-mentioned "optionally substituted hydrocarbon group" is especially preferably 5-nitro-2-pyridylamino, 5-cyano-2-pyridylamino, 2-pyrimidylamino, 2-pyrazylamino, or the like.
   A substituent for the above-mentioned "optionally substituted piperidinyl" includes C₁₋₆ alkyl; hydroxymethyl; and the "optionally substituted C₆₋₁₄ aryl" and the "optionally substituted heterocyclic group" exemplified above for the above-mentioned "amino optionally mono- or di-substituted with optionally substituted C₆₋₁₄ aryl or an optionally substituted heterocyclic group". The number of substituents is, for example, 1 to 3.
   The above-mentioned "substituent on the side chain of the amino acid" includes an optionally substituted hydrocarbon group, hydroxyl, C₁₋₁₀ alkoxy optionally substituted with 1 to 3 halogen atoms (preferably fluorine), acyl, optionally substituted amino, and the like.
   The hydrocarbon of the "optionally substituted hydrocarbon group" includes C₁₋₁₀ alkyl, C₃₋₁₂ cycloalkyl, C₂₋₁₀ alkenyl, C₃₋₁₂ cycloalkenyl, and the like.
   The hydrocarbon may be optionally substituted with 1 to 3 substituents at substitutable positions, and such substituents include amino, C₁₋₆ alkyl-carbonylamino (preferably acetylamino), hydroxy, C₁₋₆ alkoxy, heterocyclic groups (preferably pyridyl), and the like.
   The above-mentioned "acyl" is preferably optionally substituted nitrogen-containing heterocyclic-carbonyl. The "optionally substituted nitrogen-containing heterocycle" includes a nitrogen-containing heterocycle (preferably pyridine, pyridazine, pyrimidine, pyrazine, imidazole, pyrazole, thiazole, isothiazole, oxazole, isoxazole, or the like) optionally substituted with 1 to 3 substituents selected from halogen (preferably fluorine, chlorine, bromine), cyano, nitro, C₁₋₆ alkyl optionally substituted with 1 to 3 halogen atoms (preferably fluorine) (e.g. trifluoromethyl), C₁₋₆alkoxy, amino optionally mono- or di-substituted with C₁₋₆alkyl, hydroxy, carboxyl and C₁₋₆ alkyl-oxycarbonyl, and the like.
   A substituent for the above-mentioned "optionally substituted amino" includes C₁₋₆ alkyl optionally substituted with 1 to 3 substituents selected from carboxyl, carbamoyl, C₁₋₆ alkyl-oxycarbonyl and a nitrogen-containing heterocyclic group (preferably pyridyl), and the like.
   Such a substituent may optionally bind to hydroxyl, carboxyl, amino, or the like on the side chain of the amino acid.
   A salt of the compound of the formula (II) includes salts similar to a salt of the compound of the formula (I).
   The compound of the formula (II) may be anhydrous or hydrous, and further may be a prodrug.
   Preferred examples of the compound of the formula (II) include N-(N'-substituted glycyl)-2-cyano-pyrrolidine derivatives such as (2S)-1-{{{2-[(5-cyanopyridin-2-yl)amino]ethyl}amino}acetyl}-2-cyano-pyrrolidine (DPP-728) (as disclosed in WO98/19998) of the formula: 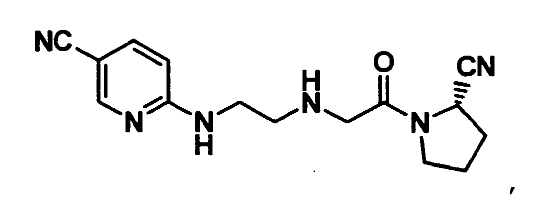 (2S)-1-{[(3-hydroxy-1-adamantyl)amino]acetyl}-2-cyanopyrrolidine (LAF238) (as disclosed in WO00/34241) of the formula: 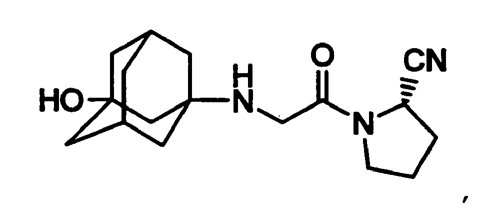 (2S)-1-{{{2-[(1-pyrimidin-2-ylpiperidin-4-yl)amino]acetyl}-2-cyano-pyrrolidine (as disclosed in WO02/30890),
   (2S)-1-{{{2-[(pyrazin-2-yl)amino]ethyl}amino}acetyl}-2-cyano-pyrrolidine, and (S)-1-{1-[5-(N,N-dimethylaminosulfonyl)-2-pyridylamino]-2-methyl-2-propylamino}acetyl-2-pyrrolidinecarbonitrile (K-361) (as disclosed in WO02/51836);
   thiazolidine or pyrrolidine derivatives such as L-threo-isoleucyl thiazolidine (P32/98) of the formula: 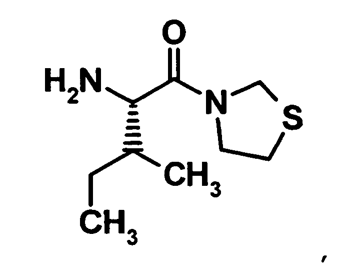 L-allo-isoleucyl thiazolidine, L-threo-isoleucyl pyrrolidine, L-allo-isoleucyl pyrrolidine and L-valyl pyrrolidine (as disclosed in WO01/72290 etc.);
   the compound (PT-100) of the formula: 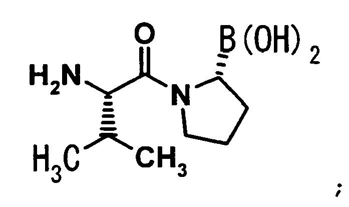 and
   the compound (P93/01) of the formula: 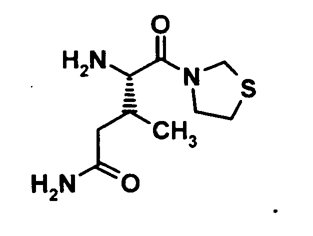
(3) N-substituted 2-cyanopyrrole and 2-cyanopyrroline derivatives as disclosed in WO01/55105, preferably, (S,S)-1-(2-amino-3,3-dimethylbutyryl)-2,5-dihydro-1H-pyrrol-2-carbonitrile.
(4) Heterocyclic compounds as disclosed in WO02/02560, preferably, 7-benzyl-8-[6-(hydroxymethyl)-1,4-diazepan-1-yl]-1,3-dimethyl-3,7-dihydropurine-2,6-dione.
(5) Pyrrolidine derivatives fused to cyclopropane as disclosed in WO01/68603, preferably, (1S,3S,5S)-2-[(2S)-2-amino-3,3-dimethylbutyryl]-3-cyano-2-azabicyclo[3.1.0]hexane.
(6) Proline derivatives as disclosed in WO02/14271, preferably, (2S)-1-[(2S,4S)-4-(3-chloro-4-cyanophenyl)amino-2-pyrrolidinylcarbonyl]-2-cyanopyrrolidine.
(7) Cyanopyrrolidine derivatives as disclosed in WO02/38541, preferably, (2S,4S)-1-[(2S,3S)-2-amino-3-methyl-pentanoyl]-2-cyano-4-fluoropyrrolidine, (2S,4S)-2-cyano-4-fluoro-1-[(1-hydroxymethyl)cyclopentylamino]acetylpyrrolidine, and (2S,4S)-2-cyano-4-fluoro-1-(1-hydroxy-3-adamantylamino)acetylpyrrolidine.
(8) Compounds as disclosed in WO02/02560, WO 03/055881, WO 03/040174, WO 03/037327, WO 03/035057, WO 03/035067, WO 03/024942, WO 03/024965, WO 03/004498, WO 03/004496, WO 03/000250, WO 03/002530, WO 03/002531, WO 03/002553, WO 03/000180, WO 03/000181, EP 1258476, WO 0251836, WO 02/68420, US 6432969,etc.; P93/01 and the like.
(9) The compound of the formula: 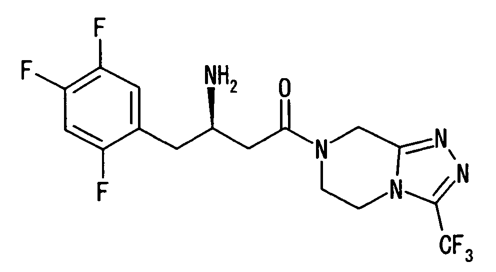 and a salt thereof (e.g. hydrochloride, phosphate) (MK-0431).
(10) The compound (BMS-477118) of the formula: 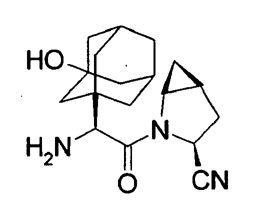
(11) A compound of the formula: 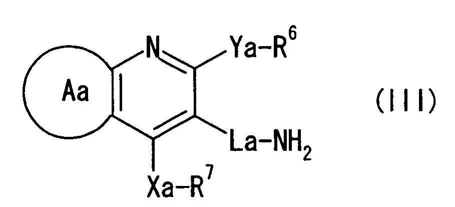
wherein, the ring Aa is an optionally substituted 5 to 10-membered aromatic ring,
R⁶ and R⁷ are the same or different and are an optionally substituted hydrocarbon group or an optionally substituted heterocyclic group,
Xa and Ya are the same or different and are a bond, -O-, - S-, -SO-, -SO₂- or -NR⁸- (wherein R⁸ is a hydrogen atom or an optionally substituted hydrocarbon group),and La is a divalent hydrocarbon group, or a salt thereof, as disclosed in WO2004/014860.

A salt of the compound of the formula (III) includes salts similar to a salt of the compound of the formula (I).

The compound of the formula (III) may be anhydrous or hydrous, and further may be a prodrug.

Preferred examples of the compound of the formula (III) include the following compounds.

### (Compound III-a)

A compound wherein the ring Aa is a benzene ring optionally substituted with 1 or 2 substituents selected from
1) halogen (e.g. fluorine, chlorine, bromine, iodine, or the like);
2) nitro;
3) cyano;
4) C₁₋₃ alkylenedioxy (e.g. methylenedioxy);
5) C₁₋₁₀ alkyl (e.g. methyl, ethyl, propyl, butyl, pentyl) or C₂₋₁₀ alkenyl (e.g. ethenyl, 3-butenyl), each of which may be optionally substituted with 1 to 3 substituents selected from halogen, hydroxyl, carboxyl, C₂₋₈ alkoxycarbonyl (e.g. ethoxycarbonyl), carbamoyl, cyano, amino, C₂₋₈ alkylcarbonylamino (e.g. acetylamino, isobutanoylamino), C₂₋₈ alkoxycarbonylamino (e.g. methoxycarbonylamino, ethoxycarbonylamino), C₁₋₈ alkylsulfonylamino (e.g. methylsulfonylamino), C₂₋₈ alkylcarbamoylamino (e.g. methylcarbamoylamino), carboxyl-C₁₋₆ alkylthio (e.g. carboxylmethylthio), C₂₋₈ alkoxycarbonyl-C₁₋₆ alkylthio (e.g. ethoxycarbonylmethylthio), and carbamoyl-C₁₋₆ alkylthio (e.g. carbamoylmethylthio);
6) optionally substituted hydroxyl [e.g. C₁₋₁₀ alkoxy (e.g. methoxy, ethoxy, propoxy, isopropoxy, butoxy, isobutoxy, pentyloxy), C₃₋₁₀ cycloalkyloxy (e.g. cyclopentyloxy) or C₇₋₁₃ aralkyloxy (e.g. benzyloxy), each of which may be optionally substituted with 1 to 3 substituents selected from halogen; C₁₋₃ alkoxy optionally substituted with 1 or 2 substituents selected from carboxyl and C₂₋₅ alkoxycarbonyl (e.g. tert-butoxycarbonyl)(e.g. methoxy, carboxylmethoxy, tert-butoxycarbonylmethoxy); C₂₋₅ alkoxycarbonyl (e.g. methoxycarbonyl, ethoxycarbonyl); C₂₋₅ alkylcarbonyl (e.g. pivaloyl); cyano; carbamoyl optionally substituted with 1 or 2 substituents selected from C₁₋₁₀ alkyl (e.g. methyl, ethyl, propyl, isopropyl) and C₁₋₁₀ alkylsulfonyl (e.g. methylsulfonyl); hydroxyl; carboxyl; amino; C₂₋₅ alkylcarbonylamino (e.g. acetylamino); an aromatic heterocyclic group (e.g. furyl, thienyl, oxazolyl, thiazolyl, isoxazolyl, tetrazolyl, oxadiazolyl, thiadiazolyl) optionally substituted with 1 to 3 substituents selected from C₁₋₆ alkyl (e.g. methyl, ethyl) and C₂₋₈ alkoxycarbonyl (e.g. methoxycarbonyl, ethoxycarbonyl); and C₃₋₁₀ cycloalkyl (e.g. cyclopropyl, cyclohexyl); or hydroxyl];
7) acyl [e.g. formyl, carboxyl, C₁₋₆ alkyl-carbonyl (e.g. acetyl), C₁₋₆ alkoxy-carbonyl (e.g. methoxycarbonyl), carbamoyl, aminocarbamoyl, hydroxycarbamoyl, mono- or di-(C₁₋₆ alkyl optionally substituted with 1 to 3 substituents selected from halogen and C₁₋₆ alkoxy-carbonyl (e.g. ethoxycarbonyl))-carbamoyl (e.g. methylcarbamoyl, ethylcarbamoyl, propylcarbamoyl, dimethylcarbamoyl, trifluoroethylcarbamoyl, ethoxycarbonylmethylcarbamoyl), C₃₋₁₀ cycloalkyl-carbamoyl (e.g. cyclopropylcarbamoyl), C₇₋₁₃ aralkyl-carbamoyl (e.g. benzylcarbamoyl), nitrogen-containing heterocyclic-carbonyl optionally substituted with hydroxyl (e.g. pyrrolidinylcarbonyl, piperidinocarbonyl), C₁₋₆ alkylsulfonyl (e.g. methylsulfonyl), C₁₋₆ alkylsulfinyl (e.g. methylsulfinyl), thiocarbamoyl];
8) optionally substituted amino [e.g. amino, mono- or di-C₂₋₁₀ alkylcarbonylamino (e.g. acetylamino, propionylamino, isobutanoylamino, isopentanoylamino), C₁₋₁₀ alkoxy-carbonylamino (e.g. methoxycarbonylamino), carbamoylamino, mono- or di-C₁₋₁₀ alkyl-carbamoylamino (e.g. methylcarbamoylamino, dimethylcarbamoylamino), C₆₋₁₄ aryl-carbonylamino (e.g. benzoylamino), C₃₋₁₀ cycloalkyl-carbonylamino (e.g. cyclopentylcarbonylamino), C₇₋₁₃ aralkyloxy-carbonylamino (e.g. benzyloxycarbonylamino), mono- or di-C₁₋₁₀ alkylsulfonylamino (e.g. methylsulfonylamino, dimethylsulfonylamino), C₆₋₁₄ arylsulfonylamino (e.g. phenylsulfonylamino), C₁₋₆ alkoxy-carbamoylamino (e.g., methoxycarbamoylamino), carbamoyl-C_{1- 10} alkylamino (e.g. carbamoylmethylamino), C₂₋₅ alkoxycarbonyl-C₁₋₁₀ alkylamino (e.g. methoxycarbonylmethylamino, tert-butoxycarbonylmethylamino)];
9) optionally substituted C₃₋₁₀ cycloalkyl [e.g. C₃₋₁₀ cycloalkyl (e.g. cyclopropyl, cyclobutyl) optionally substituted with 1 to 3 substituents selected from C₁₋₆ alkyl optionally substituted with 1 to 3 halogen atoms (e.g. methyl, trifluoromethyl), carboxyl, C₂₋₈ alkoxycarbonyl (e.g. methoxycarbonyl, ethoxycarbonyl, isopropoxycarbonyl), cyano, carbamoyl, amino, mono- or di-C₂₋₁₀ alkylcarbonylamino (e.g. acetylamino, isopentanoylamino), C₁₋₁₀ alkoxy-carbonylamino (e.g. methoxycarbonylamino), carbamoylamino, mono- or di-C₁₋₁₀ alkyl-carbamoylamino (e.g. methylcarbamoylamino, dimethylcarbamoylamino), C₆₋₁₄ aryl-carbonylamino (e.g. benzoylamino), C₃₋₁₀ cycloalkyl-carbonylamino, C₇₋₁₃ aralkyloxy-carbonylamino, mono- or di-C₁₋₁₀ alkylsulfonylamino (e.g. methylsulfonylamino, dimethylsulfonylamino), C₆₋₁₄ arylsulfonylamino, and C₁₋₆ alkoxy-carbamoylamino (e.g. methoxycarbamoylamino)];
10) C₆₋₁₄ aryl (e.g. phenyl);
11) optionally substituted thiol [e.g. C₁₋₁₀ alkylthio (e.g. methylthio) optionally substituted with carbamoyl];
12) an optionally substituted heterocyclic group [e.g. an aromatic heterocyclic group (preferably, furyl, thienyl, oxazolyl, oxadiazolyl, thiazolyl, tetrazolyl, pyridyl, pyrrolyl, triazolyl) or a nonaromatic heterocyclic group (preferably, dioxoisoindol-2-yl; 5-oxooxadiazol-3-yl; 5-oxothiadiazol-3-yl; 3-oxopiperazin-1-yl; 2,3-dioxopiperazin-1-yl; 2,5-dioxopiperazin-1-yl), each of which may be optionally substituted with 1 or 2 substituents selected from C₁₋₆ alkyl optionally substituted with 1 to 3 halogen atoms (e.g. methyl, trifluoromethyl), carboxyl, C₂₋₈ alkoxycarbonyl (e.g. methoxycarbonyl, ethoxycarbonyl, isopropoxycarbonyl, tert-butoxycarbonyl), cyano, carbamoyl, amino, mono- or di-C₂₋₁₀ alkylcarbonylamino (e.g. acetylamino, isopentanoylamino), C₁₋₁₀ alkoxy-carbonylamino (e.g. methoxycarbonylamino), carbamoylamino, mono- or di-C₁₋₁₀ alkyl-carbamoylamino (e.g. methylcarbamoylamino, dimethylcarbamoylamino), C₆₋₁₄ aryl-carbonylamino (e.g. benzoylamino), C₃₋₁₀ cycloalkyl-carbonylamino, C₇₋₁₃ aralkyloxy-carbonylamino, mono- or di-C₁₋₁₀ alkylsulfonylamino (e.g. methylsulfonylamino, dimethylsulfonylamino), C₆₋₁₄ arylsulfonylamino, C₁₋₆ alkoxy-carbamoylamino (e.g. methoxycarbamoylamino), C₂₋₅ alkylcarbonyl (e.g. acetyl) and carbamoyl-C₁₋₆ alkyl (e.g. carbamoylmethyl)]; and
13) amidino;
   R⁶ is C₃₋₁₀ alkyl (preferably isobutyl) or C₄₋₁₀ cycloalkylalkyl (preferably cyclopropylmethyl);
   R⁷ is C₁₋₁₀ alkyl (e.g. methyl, ethyl, propyl, isopropyl, butyl, isobutyl, sec-butyl, tert-butyl, pentyl, or the like), C₆₋₁₄ aryl (e.g. phenyl or the like) or C₇₋₁₃ aralkyl (e.g. benzyl, phenethyl, naphthylmethyl or the like), each of which may be optionally substituted with 1 to 3 (preferably 1 or 2) substituents selected from halogen (e.g. fluorine, chlorine or the like), hydroxy, nitro, amino, optionally halogenated C₁₋₆ alkyl (e.g. trifluoromethyl, methyl or the like), C₁₋₆ alkoxy (e.g. methoxy or the like), an aromatic heterocyclic group (e.g. quinolyl, thienyl or the like) and C₃₋₁₀ cycloalkyl (e.g. cyclopentyl or the like);
   Xa is a bond;
   Ya is a bond; and
   La is C₁₋₁₀ alkylene.
   Among the compounds of the formula (III), especially preferred are (2E)-3-[3-(aminomethyl)-2-isobutyl-4-(4-methylphenyl)quinolin-6-yl]acrylamide;
   5-[[3-(aminomethyl)-2-isobutyl-4-(4-methylphenyl)quinolin-6-yl]oxy]pentanoic acid;
   4-[3-(aminomethyl)-2-isobutyl-4-(4-methylphenyl)quinolin-6-yl]piperazin-2-one;
   1-[3-(aminomethyl)-2-isobutyl-4-(4-methylphenyl)quinolin-6-yl]piperazin-2,5-dione; and the like.
(12) A compound of the formula: 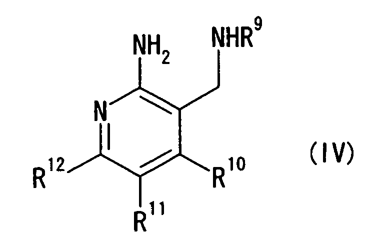 wherein R⁹ is hydrogen or lower(C₁₋₆)alkyl;
   R¹⁰ is heterocyclyl optionally mono-, di- or tri-substituted with substituents selected from lower(C₁₋₆)alkyl, lower(C₁₋₆)alkoxy, perfluoro lower(C₁₋₆)alkyl, amino and halogen; or aryl optionally mono-, di- or tri-substituted with substituents selected from halogen, lower(C₁₋₆)alkyl, lower (C₁₋₆)alkoxy, amino and perfluoro lower(C₁₋₆)alkyl; and R¹¹ and R¹², together with the carbon atoms to which they are bound, form a phenyl ring (said phenyl ring may be optionally mono-, di- or tri-substituted with substituents selected from halogen, lower(C₁₋₆)alkyl, perfluoro lower(C₁₋₆)alkyl and lower(C₁₋₆)alkoxy), or a 5-, 6- or 7-membered saturated ring (said saturated ring may optionally contain heteroatom(s) selected from O, N and S; may be optionally mono-, di- or tri-substituted with substituents selected from halogen, lower(C₁₋₆)alkyl, perfluoro lower(C₁₋₆)alkyl and lower(C₁₋₆)alkoxy; and may be optionally ortho-fused to a 5- or 6-membered aromatic ring (said aromatic ring may optionally contain heteroatom(s) selected from O, N and S; and may be optionally mono-, di- or tri-substituted with substituents selected from halogen, lower(C₁₋₆)alkyl, perfluoro lower(C₁₋₆)alkyl and lower(C₁₋₆)alkoxy)), or a salt thereof, as disclosed in WO03/068748.
   A salt of the compound of the formula (IV) includes salts similar to a salt of the compound of the formula (I).
   The compound of the formula (IV) may be anhydrous or hydrous, and further may be a prodrug.
(13) A compound of the formula: 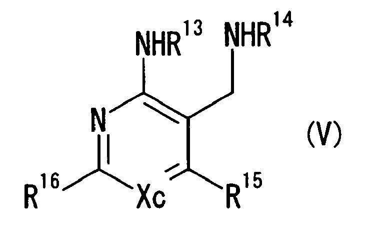 wherein Xc is N or C-R¹⁷;
   R¹³ and R¹⁴ are independently hydrogen or lower(C₁₋₆)alkyl;
   R¹⁵ is heterocyclyl optionally mono-, di- or tri-substituted with substituents selected from lower(C₁₋₆)alkyl, perfluoro lower(C₁₋₆)alkyl, amino, lower(C₁₋₆)alkoxy and halogen; or aryl optionally mono-, di- or tri-substituted with substituents selected from halogen, lower(C₁₋₆)alkyl, amino, lower(C₁₋₆)alkoxy and perfluoro lower(C₁₋₆)alkyl;
   R¹⁶ is lower (C₁₋₆)alkyl; lower (C₁₋₆)alkoxy; lower(C₁₋₆) alkylthio; heterocyclyl optionally mono-, di- or tri-substituted with substituents selected from lower(C₁₋₆)alkyl, lower(C₁₋₆)alkoxy, perfluoro lower(C₁₋₆)alkyl, amino and halogen; aryl optionally mono-, di- or tri-substituted with substituents selected from lower(C₁₋₆)alkyl, lower(C₁₋₆)alkoxy, halogen, amino and perfluoro lower(C₁₋₆)alkyl; aryloxy-lower(C₁₋₆)alkyl or cycloalkyl; and
   R¹⁷ is hydrogen or lower(C₁₋₆)alkyl, or a salt thereof, as disclosed in WO03/068757.

A salt of the compound of the formula (V) includes salts similar to a salt of the compound of the formula (I).

The compound of the formula (V) may be anhydrous or hydrous, and further may be a prodrug.

As the agent for treating diabetes with sulfonylurea secondary failure of the present invention (hereinafter, sometimes abbreviated to the agent of the present invention), a DPP-IV inhibitor as it is or as a pharmaceutical composition prepared by mixing with a pharmacologically acceptable carrier and the like can be administered to a mammal (e.g. human, mouse, rat, rabbit, dog, cat, cow, horse, pig, monkey).

The amount of a DPP-IV inhibitor contained in the agent of the present invention varies on the type of the DPP-IV inhibitor, the size of the agent, and the like. For example, it is 1 to 90% by weight, preferably 5 to 80 % by weight.

The above-mentioned pharmacologically acceptable carrier includes various organic or inorganic carriers that are conventionally used as materials for drug formulation. The carrier is incorporated into a solid preparation as an excipient, a lubricant, a binder, a disintegrant or the like; or into a liquid preparation as a solvent, a solubilizing agent, a suspending agent, an isotonicity, a buffering agent, a soothing agent or the like. Additives for drug formulation such as an antiseptic, an antioxidant, a colorant, a sweetening agent and the like may be also used, if necessary.

Preferred examples of an excipient include lactose, saccharose, D-mannitol, D-sorbitol, starch, pregelatinized starch, dextrin, crystalline cellulose, low-substituted hydroxypropylcellulose, carboxymethylcellulose sodium, acacia, pullulan, light anhydrous silicic acid, synthetic aluminum silicate, magnesium aluminometasilicate, and the like.

Preferred examples of a lubricant include magnesium stearate, calcium stearate, talc, colloidal silica, and the like.

Preferred examples of a binder include pregelatinized starch, sucrose, gelatin, acacia, methylcellulose, carboxymethylcellulose, carboxymethylcellulose sodium, crystalline cellulose, saccharose, D-mannitol, trehalose, dextrin, pullulan, hydroxypropylcellulose, hydroxypropylmethylcellulose, polyvinylpyrrolidone, and the like.

Preferred examples of a disintegrant include lactose, saccharose, starch, carboxymethylcellulose, carboxymethylcellulose calcium, croscarmellose sodium, carboxymethylstarch sodium, light anhydrous silicic acid, low-substituted hydroxypropylcellulose, and the like.

Preferred examples of a solvent include water for injection, physiological saline, Ringer's solution, alcohol, propylene glycol, polyethylene glycol, sesame oil, corn oil, olive oil, cotton seed oil, and the like.

Preferred examples of a solubilizing agent include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate, and the like.

Preferred examples of a suspending agent include surfactants such as stearyl triethanolamine, sodium laurylsulfate, laurylaminopropionic acid, lecithin, benzalkonium chloride, benzethonium chloride and glyceryl monostearate; hydrophilic polymers such as polyvinyl alcohol, polyvinylpyrrolidone, carboxymethylcellulose sodium, methylcellulose, hydroxymethylcellulose, hydroxyethylcellulose and hydroxyproylcellulose; polysorbates, polyoxyethylene hydrogenated caster oil, and the like.

Preferred examples of an isotonicity include sodium chloride, glycerin, D-mannitol, D-sorbitol, glucose, and the like.

Preferred examples of a buffering agent include buffers of phosphate, acetate, carbonate and citrate, and the like.

Preferred examples of a soothing agent include benzyl alcohol, and the like.

Preferred examples of an antiseptic include p-hydroxybenzoic acid esters, chlorobutanol, benzyl alcohol, phenethyl alcohol, dehydroacetic acid, sorbic acid, and the like.

Preferred examples of an antioxidant include sulfite, ascorbate, and the like.

Preferred examples of a colorant include water-soluble food tar dyes (e.g. food dyes such as food red No.2 and No.3, food yellow No.4 and No.5, and food blue No.1 and No.2), water-insoluble lake dyes (e.g. aluminum salts of the above-mentioned water-soluble food tar dyes), natural dyes (e.g. β-carotene, chlorophyll, red ocher, yellow iron sesquioxide), and the like.

Preferred examples of a sweetening agent include saccharin sodium, dipotassium glycyrrhizinate, aspartame, stevia, and the like.

The dosage forms of the agent of the present invention include oral preparations such as tablets (including sublingual tablets and intraorally disintegrating tablets), capsules (including soft capsules and microcapsules), granules, powders, troches, syrups, emulsions and suspensions; and parenteral preparations such as injections (e.g. subcutaneous injections, intravenous injections, intramuscular injections, intraperitoneal injections), external preparations (e.g. nasal preparations, percutaneous preparations, ointments and the like), suppositories (e.g. rectal suppositories and vaginal suppositories), pellets, preparations for drip infusion, eye drops, and preparations for transpulmonary administration (inhalants and the like). These preparations can be orally or parenterally safely administered. These preparations may be controlled-release preparations such as immediate-release preparations or sustained-release preparations (for example, sustained-release microcapsules).

The agent of the present invention can be manufactured with a conventional method in the art, for example, a method described in the Japanese Pharmacopoeia or the like. Hereinafter, specific methods for manufacture of the preparations are explained in detail.

An oral preparation, for example, is manufactured by adding an excipient (e.g. lactose, saccharose, starch, D-mannitol or the like), a disintegrant (e.g. carboxymethylcellulose calcium or the like), a binder (e.g. pregelatinized starch, acacia, carboxymethylcellulose, hydroxypropylcellulose, polyvinylpyrrolidone or the like) or a lubricant (e.g. talc, magnesium stearate, polyethylene glycol 6000 or the like) to the active ingredient, compression molding the mixture, and then, if necessary, coating the resulting molded product with a coating base according to a method per se known for the purpose of masking of taste or making the preparation enteric or sustained-release.

The coating base includes a sugar coating base, a water-soluble film coating base, an enteric film coating base, a sustained-release film coating base, and the like.

As a sugar coating base, saccharose may be used or may be used in combination with 1 or 2 selected from talc, precipitated calcium carbonate, gelatin, acacia, pullulan, carnauba wax and the like.

A water-soluble film coating base includes cellulose polymers such as hydroxypropylcellulose, hydroxypropylmethylcellulose, hydroxyethylcellulose and methylhydroxyethylcellulose; synthetic polymers such as polyvinylacetal diethylaminoacetate, aminoalkylmethacrylate copolymer E [Eudragit E (trade name), Roehm Pharma] and polyvinylpyrrolidone; polysaccharides such as pullulan; and the like.

An enteric film coating base includes cellulose polymers such as hydroxypropylmethylcellulose phthalate, hydroxypropylmethylcellulose acetate succinate, carboxymethylethylcellulose, and cellulose acetate phthalate; acrylate polymers such as methacrylate copolymer L [Eudragit L (trade name), Roehm Pharma], methacrylate copolymer LD [Eudragit L-30D55 (trade name), Roehm Pharma], and methacrylate copolymer S [Eudragit S (trade name), Roehm Pharma]; natural products such as shellac; and the like.

A sustained-release film coating base includes cellulose polymers such as ethyl cellulose; acrylate polymers such as aminoalkylmethacrylate copolymer RS [Eudragit RS (trade name), Roehm Pharma] and ethyl acrylate/methyl methacrylate copolymer suspension [Eudragit NE (trade name), Roehm Pharma]; and the like.

Two or more of the above-mentioned coating bases may be mixed at an appropriate proportion and then used. In a coating step, a light-blocking agent such as titanium dioxide or iron sesquioxide may be also used.

An injection is manufactured by dissolving, suspending or emulsifying the active ingredient in a water-soluble solvent (e.g. distilled water, physiological saline, Ringer's solution, or the like) or an oily solvent (e.g. a vegetable oil such as olive oil, sesame oil, cotton seed oil or corn oil, propylene glycol, or the like) together with a dispersing agent (e.g. Polysorbate 80, polyoxyethylene hydrogenated caster oil 60, polyethylene glycol, carboxymethylcellulose, sodium arginate, or the like), a preservative (e.g. methylparaben, propylparaben, benzyl alcohol, chlorobutanol, phenol, or the like), an isotonicity (e.g. sodium chloride, glycerin, D-mannitol, D-sorbitol, glucose, or the like) and the like. In this process, additives such as a solubilizing agent (e.g. sodium salicylate, sodium acetate, or the like), a stabilizer (e.g. human serum albumin, or the like), a soothing agent (e.g. benzyl alcohol, or the like) and the like may be used, if necessary.

Among the above-mentioned various preparations, preferred are oral preparations which are excellent in convenience in use or compliance.

In the specification, the "sulfonylurea secondary failure" of "diabetes with sulfonylurea secondary failure" means a condition in which "a drug that promotes insulin secretion from pancreatic β-cells by binding to the sulfonylurea receptor 1 (SUR1) constituting the ATP-sensitive K⁺ channel (hereinafter sometimes abbreviated to K_{ATP} channel) of pancreatic β-cells to close the K_{ATP} channel and cause depolarization on the cellular membrane [e.g. sulfonylurea compound (sulfonylurea antidiabetic agent), fast-acting insulin secretagogue]" has become ineffective in lowering the blood sugar level due to repeated or long-term (e.g. for 2 or more weeks, preferably for 4 or more weeks) administration of said drug.

The above-mentioned sulfonylurea compound includes compounds having a sulfonylurea skeleton and the derivatives thereof, for example, tolbutamide, glibenclamide, gliclazide, chlorpropamide, tolazamide, acetohexamide, glyclopyramide, glimepiride, glipizide, glybuzole, and the like.

The fast-acting insulin secretagogue includes compounds that promote insulin secretion from pancreatic β-cells as in the case of sulfonylurea compounds, although do not have a sulfonylurea skeleton, for example, glinide compounds such as repaglinide, senaglinide, nateglinide, mitiglinide or its calcium salt hydrate, and the like.

Sulfonylurea secondary failure may be ascribable to either of a sulfonylurea compound and a fast-acting insulin secretagogue.

Diabetes of "diabetes with sulfonylurea secondary failure" includes type 1 diabetes, type 2 diabetes, gestational diabetes, impaired glucose tolerance (IGT), impaired fasting glucose (IFG), impaired fasting glycemia (IFG), diabetic complications [e.g. neuropathy, nephropathy, retinopathy, cataract, macroangiopathy, osteopenia, diabetic hyperosmolar coma, infections (e.g., respiratory tract infection, urinary tract infection, alimentary canal infection, dermal soft tissue infection, inferior limb infection, etc.), diabetic gangrene, xerostomia, hypacusis, cerebrovascular disorder, peripheral blood circulation disorder] and the like. Among these, type 2 diabetes is preferable.

The agent for treating diabetes with sulfonylurea secondary failure of the present invention shows excellent insulin-secreting and hypoglycemic effects on even diabetic patients on whom a sulfonylurea compound or a fast-acting insulin secretagogue has no insulin-secreting effect and therefore no sufficient hypoglycemic effect.

A dose of the agent of the present invention varies on a subject to be administered, an administration route, targeted disease and the like. For example, when the agent of the present invention is orally administered to an adult diabetic patient, the unit dose is usually about 0.01 to 100 mg/kg bodyweight, preferably 0.05 to 30 mg/kg bodyweight, and more preferably 0.1 to 10 mg/kg bodyweight of the active ingredient, a DPP-IV inhibitor. Preferably, the unit dose is administered once or twice a day.

The agent of the present invention can be used in combination with a drug such as a diabetic treating agent, a diabetic complication treating agent, a hyperlipemia treating agent, a hypotensive drug, an anti-obesity drug, a diuretic, an antithrombotic drug or the like (hereinafter abbreviated to a concomitant drug), for the purpose of enhancing the efficacy of or reducing the dose of the agent of the present invention. In this case, the timing for administering the agent of the present invention and a concomitant drug is not restricted. They may be administered simultaneously or separately at an interval of time. Alternatively, the agent of the present invention and a concomitant drug may be administered as two separate preparations containing the respective active ingredients or as a single preparation containing both the active ingredients.

A dose of a concomitant drug can be selected appropriately based on the clinical dose. The combination ratio between the agent of the present invention and a concomitant drug can be selected appropriately depending on a subject to be administered, an administration route, disease to be treated, symptoms and a combination of drugs. In the case where a subject to be administered is a human, 0.01 to 100 parts by weight of a concomitant drug may be used per 1 part by weight of the agent of a DPP-IV inhibitor, the active ingredient of the present invention.

The above-mentioned diabetic treating agent includes insulin preparations (e.g., animal-derived insulin preparations extracted from bovine or swine pancreas; human insulin preparations which are synthesized with genetic engineering using Escherichia coli or yeast; insulin zinc; protamine insulin zinc; insulin fragments or derivatives (for example, INS-1) etc.), insulin sensitizers (e.g. pioglitazone or a salt thereof (preferably hydrochloride), rosiglitazone or a salt thereof (preferably maleate), GI-262570, reglixane (JTT-501), netoglitazone (MCC-555), KRP-297, rivoglitazone (CS-011), FK-614, ragaglitazar (NN-622), tesaglitazar (AZ-242), muraglitazar (BMS-298585), EML-16336, compounds described in WO99/58510 (e.g. (E)-4-[4-(5-methyl-2-phenyl-4-oxazolylmethoxy)benzyloxyimino]-4-phenylbutyric acid), compounds described in WO01/38325, BM-13-1258, LM-4156, MBX-102, LY-519818, MX-6054, LY-510929, balaglitazone (NN-2344), T-131 or a salt thereof, THR-0921), PPARγ agonists, PPARγ antagonists, PPARγ/α dual agonists, α-glucosidase inhibitors (e.g., voglibose, acarbose, miglitol, emiglitate), biguanide agents [e.g., phenformin, metformin, buformin, or their salts (e.g., hydrochloride, fumarate, succinate)], GLP-1 receptor agonists [e.g. GLP-1, NN-2211, AC-2993 (exendin-4), BIM-51077, Aib(8,35)hGLP-1(7,37)NH₂], amylin agonists (e.g., pramlintide), phosphotyrosine phosphatase inhibitors (e.g., sodium vanadate), β3 agonists (e.g. CL-316243, SR-58611-A, UL-TG-307, SB-226552, AJ-9677, BMS-196085, AZ40140), glyconeogenesis inhibitors (e.g., glycogen phosphorylase inhibitors, glucose-6-phosphatase inhibitors, glucagon antagonists, somatostatin receptor agonists), sodium-glucose cotransporter (SGLT) inhibitors (e.g., T-1095), 11β-hydroxysteroiddehydrogenase inhibitors (e.g., BVT-3498 etc.), adiponectin or agonists thereof, IKK inhibitors (e.g., AS-2868 etc.), leptin sensitivity-improving agents, somatostatin receptor agonists (e.g., compounds described in WO01/25228, WO03/42204, WO98/44921, WO98/45285, WO99/22735 etc.), glucokinase activators (e.g., Ro-28-1675) and the like.

The diabetic complication treating agent includes aldose reductase inhibitors (e.g., tolrestat, epalrestat, zenarestat, zopolrestat, minalrestat, fidarestat (SNK-860), CT-112), neurotrophic factors and enhancers therefor [e.g., NGF, NT-3, BDNF, neurotrophin production/secretion promotors described in WO01/14372 (e.g., 4-(4-chlorophenyl)-2-(2-methyl-1-imidazolyl)-5-(3-(2-methylphenoxy)propyl)oxazole) etc.], neural regeneration promotors (e.g. Y-128), PKC inhibitors (e.g., ruboxistaurin mesylate; LY-333531), AGE inhibitors (e.g., ALT946, pimagedine, piratoxathin, N-phenacylthiazolium bromide (ALT766), EXO-226), reactive oxygen scavengers (e.g., thioctic acid), cerebral vasodilators (e.g., tiapride, mexiletine), somatostatin receptor agonists (e.g., BIM23190), apoptosis signal regulating kinase-1 (ASK-1) inhibitors, and the like.

The hyperlipemia treating agent includes statin compounds that acts as cholesterol synthesis inhibitors (e.g., cerivastatin, pravastatin, simvastatin, lovastatin, atorvastatin, fluvastatin, pitavastatin, rosuvastatin or their salts (sodium salts, calcium salts)), squalene synthase inhibitors (e.g., compounds described in WO97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepine-3-yl]acetyl]piperidine-4-acetic acid, etc.), fibrate compounds (e.g., benzafibrate, clofibrate, simfibrate, clinofibrate), ACAT inhibitors (e.g., avasimibe, eflucimibe), anion-exchange resins (e.g., cholestyramine), probucol, nicotinic acid drugs (e.g., nicomol, niceritrol), ethyl icosapentate, phytosterol (e.g., soysterol, γ-oryzanol), and the like.

The hypotensive drug includes angiotensin converting enzyme inhibitors (e.g., captopril, enalapril, delapril), angiotensin II antagonists (e.g., candesartan, cilexetil, losartan, eprosartan, valsartan, telmisartan, irbesartan, tasosartan, olmesartan medoxomil, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121), clonidine, and the like.

The anti-obesity drug includes anti-obesity drugs acting on the central nervous system [e.g., dexfenfluramine, fenfluramine, phentermine, sibutramine, amphepramone, dexanphetamine, mazindol, phenylpropanolamine, clobenzorex; MCH receptor antagonists (e.g., SB-568849, SNAP-7941, compounds described in WO01/82925 and WO01/87834 etc.); neuropeptide Y antagonists (e.g., CP-422935 etc.); cannabinoid receptor antagonists (e.g., SR-141716, SR-147778 etc.); ghrelin antagonists; 11β-hydroxysteroiddehydrogenase inhibitors (e.g., BVT-3498 etc.), etc.], pancreatic lipase inhibitors (e.g., orlistat, ATL-962), β3 agonists (e.g., CL-316243, SR-58611-A, UL-TG-307, SB-226552, AJ-9677, BMS-196085, AZ-40140), peptidic anorectics (e.g., leptin, CNTF (ciliary neurotrophic factors)), cholecystokinin agonists (e.g., lintitript, FPL-15849), and the like.

The diuretic includes xanthine derivatives (e.g., sodium salicylate theobromine, calcium salicylate theobromine), thiazide agents (e.g., ethiazide, cyclopenthiazide, trichlormethiazide, hydrochlorothiazide, hydroflumethiazide, benzylhydrochlorothiazide, penflutizide, polythiazide, methyclothiazide), anti-aldosterone drugs (e.g., spironolactone, triamterene), carbonic anhydrase inhibitors (e.g., acetazolamide), chlorobenzenesulfonamide drugs (e.g., chlorthalidone, mefruside, indapamide), azosemide, isosorbide, etacrynic acid, piretanide, bumetanide, furosemide, and the like.

The antithrombotic drug includes heparin (e.g., heparin sodium, heparin calcium, dalteparin sodium), warfarin (e.g., warfarin potassium), anti-thrombin agents (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride), and the like.

Preferred concomitant drug is an insulin preparation, an insulin sensitizer, an α-glucosidase inhibitor, a biguanide or the like.

The present invention further relates to "an insulin secretagogue for diabetic patients with sulfonylurea secondary failure which comprises a DPP-IV inhibitor".

The DPP-IV inhibitor and the diabetes with sulfonylurea secondary failure are as defined above.

The above-mentioned insulin secretagogue can be produced using a DPP-IV inhibitor and then used, similarly to the above-mentioned agent for treating diabetes with sulfonylurea secondary failure. The insulin secretagogue is useful as an agent for treating diabetes with sulfonylurea secondary failure, and shows excellent insulin-secreting and hypoglycemic effects on even diabetic patients on whom a sulfonylurea compound or a fast-acting insulin secretagogue has no insulin-secreting effect and therefore no sufficient hypoglycemic effect.

The present invention further relates to "a closer of the ATP-sensitive K⁺ channel that has become unable to be closed as a result of stimulation by a sulfonylurea receptor 1-binding compound (e.g. sulfonylurea compound, fast-acting insulin secretagogue) comprising a DPP-IV inhibitor".

The DPP-IV inhibitor, the sulfonylurea compound and the fast-acting insulin secretagogue are as defined above.

The above-mentioned closer can be produced using a DPP-IV inhibitor and then used, similarly to the above-mentioned agent for treating diabetes with sulfonylurea secondary failure. The closer is specifically useful as an agent for treating diabetes with sulfonylurea secondary failure.

The present invention is illustrated in further detail by the following Example and Experimental Examples.

In the following Example and Experimental Examples, 2-{[3-(aminomethyl)-2-isobutyl-4-phenyl-1-oxo-1,2-dihydro-6-isoquinolinyl]oxy}acetamide monohydrate is abbreviated to Compound A.

### Example 1

Compound A (150 mg), lactose (1184 mg), corn starch (360 mg), HPC-L (trade name, manufactured by Nippon Soda Co., Ltd.) (60 mg), carboxymethylcellulose calcium (trade name: ECG505, manufactured by Gotoku Chemical Company Ltd.) (60 mg), crystalline cellulose (trade name: Avicel, manufactured by Asahi Kasei Corporation) (172 mg), and magnesium stearate (14 mg) were mixed in a mortar. 200 mg of the resultant mixture was compressed using a hydraulic pump press (manufactured by Riken Seiki Co., Ltd.) to obtain a tablet with a diameter of 8 mm.

### Experimental Example 1

Streptozocin (STZ) (120 mg/kg bodyweight) was administered to a 1.5-day-old male WKY rat to obtain a type 2 diabetes model, N-STZ-1.5 rat.

N-STZ-1.5 rats (male, 24 animals) received orally glibenclamide (10 mg/kg bodyweight/day) for 4 weeks to become a model of type 2 diabetes with sulfonylurea secondary failure. Then, the rats were divided into 4 groups of Groups A to D (6 animals each). Group A (control group), Group B, Group C and Group D were treated with oral administration of a 0.5% methylcellulose suspension, glibenclamide (10 mg/kg bodyweight), nateglinide (50 mg/kg bodyweight) and Compound A (3 mg/kg bodyweight), respectively. (Hereinafter, the methylcellulose suspension, glibenclamide, nateglinide and Compound A administered to Groups A to D are sometimes collectively referred to as the test compound.)

Then, each rat received orally 1 g/kg bodyweight of glucose solution. Before administration of glucose (before and after administration of the test compound) and 10 and 60 minutes after administration of glucose solution, blood samples were taken from the caudal vein of the rat and then the plasma glucose level and the plasma insulin level were determined.

The plasma glucose level was determined by an enzymatic method using L type WakoGlu2 (trade name, Wako Pure Chemical Industries, Ltd.), and the plasma insulin level was determined by radioimmunoassay using Shionoria Insulin Kit (trade name, Shionogi & Co., Ltd.).

The increment of plasma glucose level 60 minutes after administration of glucose solution (from 0 to 60 minutes after sugar loading); the increment of plasma insulin level 10 minutes after administration of glucose solution (from before administration of the test compound to 10 minutes after sugar loading); and the increment of plasma insulin level 10 minutes after administration of glucose solution (from 0 to 60 minutes after sugar loading) are shown in Table 1, Table 2 and Table 3, respectively. The values in the tables are mean values (n=6).

**Table 1**

| Group | Increment of plasma glucose level (mg/dl) |
|---|---|
| Group A (control) | 172.72 |
| Group B (glibenclamide) | 180.65 |
| Group C (nateglinide) | 185.95 |
| Group D (Compound A) | 134.83 |

**Table 2**

| Group | Increment of plasma insulin level (µU/ml) |
|---|---|
| Group A (control) | 19.37 |
| Group B (glibenclamide) | 18.03 |
| Group C (nateglinide) | 18.76 |
| Group D (Compound A) | 44.06 |

**Table 3**

| Group | Increment of plasma insulin level (µU/ml) |
|---|---|
| Group A (control) | 24.88 |
| Group B (glibenclamide) | 22.76 |
| Group C (nateglinide | 16.78 |
| Group D (Compound A) | 46.69 |

As seen in Table 1, administration of glibenclamide (sulfonylurea compound) or nateglinide (fast-acting insulin secretagogue) to the type 2 diabetic rat with sulfonylurea secondary failure had no plasma glucose level-lowering effect, but administration of Compound A (DPP-IV inhibitor) achieved excellent plasma glucose level-lowering effect.

As seen in Table 2 and Table 3, administration of glibenclamide (sulfonylurea compound) or nateglinide (fast-acting insulin secretagogue) to the type 2 diabetic rat with sulfonylurea secondary failure had hardly plasma insulin level-elevating effect, but administration of Compound A (DPP-IV inhibitor) achieved excellent plasma insulin level-elevating effect.

### Experimental Example 2

N-STZ-1.5 rats (male, 24 animals) prepared in Experimental Example 1 were divided into 4 groups (6 animals each), of which 2 groups (hereinafter sometimes referred to as Group M) and the remaining 2 groups (hereinafter sometimes referred to as Group G) received orally a 0.5% methylcellulose suspension and glibenclamide (10 mg/kg bodyweight) respectively, for 4 weeks. After repeated administration for 4 weeks, one Group M and one Group G (control groups) received orally a 0.5% methylcellulose suspension and the other Group M and the other Group G received orally glibenclamide (10 mg/kg bodyweight).

Then, each rat received orally 1 g/kg bodyweight of glucose solution. Before administration of glucose solution and 10 and 60 minutes after the administration, blood samples were taken from the caudal vein of the rat and then the plasma glucose level and the plasma insulin level were determined.

The plasma glucose level and the plasma insulin level were determined in the same manner as in Experimental Example 1.

The increment of plasma glucose level 60 minutes after administration of glucose solution (from 0 to 60 minutes after sugar loading) and the increment of plasma insulin level 10 minutes after administration of glucose solution (from 0 to 60 minutes after sugar loading) are shown in Table 4 and Table 5, respectively. The values in the tables are mean values (n=6).

**Table 4**

| Group | Increment of plasma glucose level (mg/dl) |
|---|---|
| Group M (control) | 100.05 |
| Group M (glibenclamide) | 51.72 |
| Group G (control) | 172.72 |
| Group G (glibenclamide) | 180.65 |

**Table 5**

| Group | Increment of plasma insulin level (µU/ml) |
|---|---|
| Group M (control) | 29.25 |
| Group M (glibenclamide) | 49.70 |
| Group G (control) | 24.88 |
| Group G (glibenclamide) | 22.76 |

As seen in Table 4, in Group M, administration of glibenclamide (sulfonylurea compound) showed plasma glucose level-lowering effect, whereas in Group G, the effect was not shown. As seen in Table 5, in Group M, administration of glibenclamide (sulfonylurea compound) showed plasma insulin level-elevating effect, whereas in Group G, the effect was not shown.

That is to say, the Experimental Example confirmed that N-STZ-1.5 rats to which glibenclamide (10 mg/kg bodyweight) had been orally administered for 4 weeks were type 2 diabetic rats with sulfonylurea secondary failure.

### Industrial Applicability

The agent for treating diabetes with sulfonylurea secondary failure of the present invention shows excellent insulin-secreting and hypoglycemic effects on even diabetic patients on whom a sulfonylurea compound or a fast-acting insulin secretagogue has no insulin-secreting effect and therefore no sufficient hypoglycemic effect

## Claims

1. Dipeptidyl peptidase IV inhibitor in the treatment of diabetic patients with sulfonylurea secondary failure said diabetic patients being **characterized by** the fact that the ATP-sensitive K⁺ channel has become unable to be closed as a result of stimulation by a sulfonylurea receptor binding compound.

2. Dipeptidyl peptidase IV inhibitor for its use as claimed in claim 1 wherein the sulfonylurea receptor binding compound is a sulfonylurea compound.

3. Dipeptidyl peptidase IV inhibitor for its use as claimed in claim 1 wherein the sulfonylurea receptor binding compound is a fast acting insulin secretagogue.

4. Dipeptidyl peptidase IV inhibitor for its use as claimed in claim 1 wherein the dipeptidyl peptidase IV inhibitor is a non-peptidic compound.

5. Dipeptidyl peptidase IV inhibitor for its use as claimed in claim 1 which is (2S)-1-{[3-hydroxy-1-adamantyl)amino]acetyl}-2-cyano-pyrrolidine.

6. Dipeptidyl peptidase IV inhibitor for its use according to claim 1 which corresponds to formula: 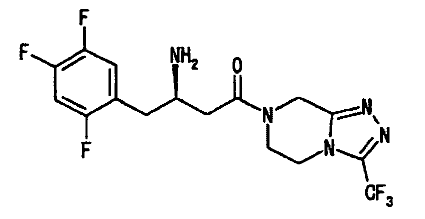 or a salt thereof.

7. Use of a Dipeptidyl peptidase IV inhibitor according to claim 1 to 6 for the manufacture of an agent for the treatment of diabetic patients with sulfonylurea secondary failure said diabetic patients being **characterized by** the fact that the ATP-sensitive K⁺ channel has become unable to be closed as a result of stimulation by a sulfonylurea receptor binding compound.

## Patentansprüche

1. Dipetidylpeptidase-IV-Inhibitor zur Verwendung in der Behandlung von Diabetespatienten mit Sekundärversagen nach Gabe von Sulfonylharnstoffen, wobei die Diabetespatienten **dadurch gekennzeichnet sind, dass** der ATP-sensitive K⁺-Kanal nicht mehr in der Lage ist, sich aufgrund der Stimulation durch eine an den Sulfonylharnstoffrezeptor bindende Verbindung zu schließen.

2. Dipetidylpeptidase-IV-Inhibitor zur Verwendung nach Anspruch 1, wobei die an den Sulfonylharnstoffrezeptor bindende Verbindung eine Sulfonylharnstoff-Verbindung ist.

3. Dipetidylpeptidase-IV-Inhibitor zur Verwendung nach Anspruch 1, wobei die an den Sulfonylharnstoffrezeptor bindende Verbindung eine schnell wirkende, die Insulinausschüttung anregende Verbindung ist.

4. Dipetidylpeptidase-IV-Inhibitor zur Verwendung nach Anspruch 1, wobei der Dipetidylpeptidase-IV-Inhibitor eine nichtpeptidische Verbindung ist.

5. Dipetidylpeptidase-IV-Inhibitor zur Verwendung nach Anspruch 1, wobei der Dipetidylpeptidase-IV-Inhibitor (2S)-1-{((3-hydroxy-1-adamantyl)amino)acetyl}-2-cyano-pyrrolidin ist.

6. Dipetidylpeptidase-IV-Inhibitor zur Verwendung nach Anspruch 1, welcher der folgenden Formel: 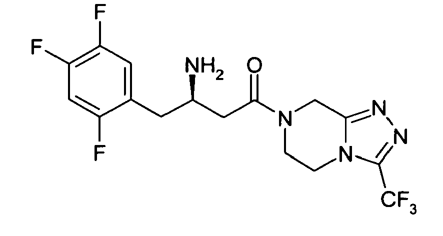 oder einem Salz davon entspricht.

7. Verwendung eines Dipetidylpeptidase-IV-Inhibitors nach Anspruch 1 bis 6 für die Herstellung von einem Mittel für die Behandlung von Diabetespatienten mit Sekundärversagen nach Gabe von Sulfonylharnstoffen, wobei die Diabetespatienten **dadurch gekennzeichnet sind, dass** der ATP-sensitive K⁺-Kanal nicht mehr in der Lage ist, sich aufgrund der Stimulation durch eine an den Sulfonylharnstoffrezeptor bindende Verbindung zu schließen.

## Revendications

1. Inhibiteur de dipeptidyl-peptidase IV, pour son utilisation dans le traitement de patients diabétiques présentant un échec secondaire aux sulfonyl-urées, lesdits patients diabétiques étant **caractérisés par** une incapacité des canaux potassiques ATP-sensibles à se fermer, résultant d'une stimulation par un composé se liant aux récepteurs de sulfonyl-urées.

2. Inhibiteur de dipeptidyl-peptidase IV pour son utilisation conforme à la revendication 1, le composé se liant aux récepteurs de sulfonyl-urées étant une sulfonyl-urée.

3. Inhibiteur de dipeptidyl-peptidase IV pour son utilisation conforme à la revendication 1, le composé se liant aux récepteurs de sulfonyl-urées étant un sécrétagogue de l'insuline à action rapide.

4. Inhibiteur de dipeptidyl-peptidase IV pour son utilisation conforme à la revendication 1, lequel inhibiteur de dipeptyl-peptidase IV est un composé non-peptidique.

5. Inhibiteur de dipeptidyl-peptidase IV pour son utilisation conforme à la revendication 1, qui est la (2S)-1-{[(3-hydroxy-adamant-1-yl)-amino]-acétyl}-2-cyano-pyrrolidine.

6. Inhibiteur de dipeptidyl-peptidase IV pour son utilisation conforme à la revendication 1, qui a pour formule : 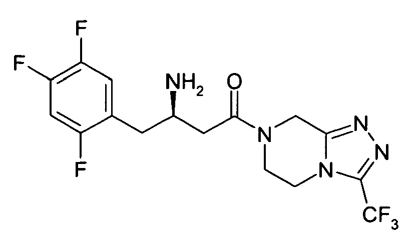 ou sel d'un tel composé.

7. Utilisation d'un inhibiteur de dipeptidyl-peptidase IV conforme à l'une des revendications 1 à 6, en vue de la fabrication d'un agent conçu pour le traitement de patients diabétiques présentant un échec secondaire aux sulfonyl-urées, lesdits patients diabétiques étant **caractérisés par** une incapacité des canaux potassiques ATP-sensibles à se fermer, résultant d'une stimulation par un composé se liant aux récepteurs de sulfonyl-urées.
